# FASCICULE DE BREVET EUROPEEN

(11) **EP 3 455 246 B1**
(45) Date de publication et mention de la délivrance du brevet: **10.03.2021**
(21) Numéro de dépôt: 17727654.0
(22) Date de dépôt: 11.05.2017
(51) Int. Cl.: C07K 14/47

(54) **MÉTHODE DE PURIFICATION DE PROTÉINES RECOMBINANTES PAR AFFINITÉ BASÉE SUR L'ACTIVITÉ LECTINIQUE DU DOMAINE CRD D'UNE GALECTINE**
VERFAHREN ZUR AFFINITÄTSREINIGUNG VON REKOMBINANTEN PROTEINEN AUF BASIS DER LECITINAKTIVITÄT DES CRD EINES GALECTINS
METHOD FOR THE AFFINITY PURIFICATION OF RECOMBINANT PROTEINS BASED ON THE LECTIN ACTIVITY OF THE CRD OF A GALECTIN

(30) Priorité: 13.05.2016 FR 1654324
(43) Date de publication de la demande: 20.03.2019
(73) Titulaire: Université de Lorraine, 54052 Nancy Cedex (FR); Centre Hospitalier Regional de Nancy, 54000 Nancy (FR)
(72) Inventeur: KRIZNIK, Alexandre, 54600 Villers-Lès-Nancy (FR); REBOUL, Pascal, 54500 Vandoevre-Lès-Nancy (FR); YELEHE-OKOUMA, Mélissa Jenner, 54000 NANCY (FR)
(74) Mandataire: Novagraaf Technologies
(86) Numéro de dépôt international: PCT/FR2017/051140
(87) Numéro de publication internationale: WO 2017/194888

(56) Documents cités:
- US-A1- 2009 280 535
- US-A1- 2012 114 674
- TAYLOR M E ET AL: "Carbohydrate-recognition domains as tools for rapid purification of recombinant eukaryotic proteins", BIOCHEMICAL JOURNAL, PORTLAND PRESS LTD, GB, vol. 274, 1 janvier 1991 (1991-01-01), pages 575-580, XP002120284, ISSN: 0264-6021
- A. S. Zdanov, J. Phan, A. G. Evdokimov, J. E. Tropea, H. K. PetersIII, R. B. Kapust, M. Li, A. Wlodawer, D. S. Waugh: "Tobacco Etch Virus Protease: Crystal Structure of the Active Enzyme and Its Inactive Mutant | SpringerLink", , vol. 29 1 janvier 2003 (2003-01-01), XP055303871, DOI: 10.1023/A:1026041223534 Extrait de l'Internet: URL:http://link.springer.com/article/10.10 23/A:1026041223534 [extrait le 2016-09-20]
- MICHELLE C MILLER ET AL: "Binding of polysaccharides to human galectin-3 at a noncanonical site in its carbohydrate recognition domain", GLYCOBIOLOGY, vol. 26, no. 1, 7 décembre 2015 (2015-12-07), pages 88-99, XP055303889, US ISSN: 0959-6658, DOI: 10.1093/glycob/cwv073
- DUMIC J ET AL: "Galectin-3: An open-ended story", BIOCHIMICA ET BIOPHYSICA ACTA (BBA) - GENERAL SUBJECTS, ELSEVIER, AMSTERDAM, NL, vol. 1760, no. 4, 1 avril 2006 (2006-04-01), pages 616-635, XP025014809, ISSN: 0304-4165, DOI: 10.1016/J.BBAGEN.2005.12.020 [extrait le 2006-04-01]
- HIRABAYASHI J ET AL: "Oligosaccharide specificity of galectins: a search by frontal affinity chromatography", BIOCHIMICA ET BIOPHYSICA ACTA (BBA) - GENERAL SUBJECTS, ELSEVIER, AMSTERDAM, NL, vol. 1572, no. 2-3, 19 septembre 2002 (2002-09-19), pages 232-254, XP004380519, ISSN: 0304-4165, DOI: 10.1016/S0304-4165(02)00311-2

### SEQUENCE LISTING

<110> Université de Lorraine
   CHRU de Nancy
   KRIZNIK, Alexandre
   REBOUL, Pascal
   YELEHE-OKOUMA, Mélissa Jenner
<120> METHODE DE PURIFICATION DE PROTEINES RECOMBINANTES PAR AFFINITE BASEE SUR L'ACTIVITE LECTINIQUE DU DOMAINE CRD DE LA GALECTINE-3 HUMAINE
<130> BNT220567PC00
<140> FR1654324
   <141> 2016-05-13
<160> 11
<170> PatentIn version 3.5
<210> 1
   <211> 157
   <212> PRT
   <213> Homo sapiens
<220>
   <221> MISC_FEATURE
   <222> (1)..(1)
   <223> Xaa est méthionine ou rien
<220>
   <221> MISC_FEATURE
   <222> (2)..(2)
   <223> Xaa est glycine, ou rien dans la version optimisée du CRDSAT
<220>
   <221> MISC_FEATURE
   <222> (36)..(36)
   <223> Xaa est arginine, ou lysine dans la version optimisée du CRDSAT
<220>
   <221> MISC_FEATURE
   <222> (152)..(152)
   <223> Xaa est alanine, ou thréonine dans la version optimisée du CRDSAT
<400> 1
   Xaa Xaa Ser Ala Thr Gly Ala Tyr Pro Ala Thr Gly Pro Tyr Gly Ala
   1               5                   10                  15
   Pro Ala Gly Pro Leu Ile Val Pro Tyr Asn Leu Pro Leu Pro Gly Gly
               20                  25                  30
   Val Val Pro Xaa Met Leu Ile Thr Ile Leu Gly Thr Val Lys Pro Asn
           35                  40                  45
   Ala Asn Arg Ile Ala Leu Asp Phe Gln Arg Gly Asn Asp Val Ala Phe
       50                  55                  60
   His Phe Asn Pro Arg Phe Asn Glu Asn Asn Arg Arg Val Ile Val Cys
   65                  70                  75                  80
   Asn Thr Lys Leu Asp Asn Asn Trp Gly Arg Glu Glu Arg Gln Ser Val
                   85                  90                  95
   Phe Pro Phe Glu Ser Gly Lys Pro Phe Lys Ile Gln Val Leu Val Glu
               100                 105                 110
   Pro Asp His Phe Lys Val Ala Val Asn Asp Ala His Leu Leu Gln Tyr
           115                 120                 125
   Asn His Arg Val Lys Lys Leu Asn Glu Ile Ser Lys Leu Gly Ile Ser
       130                 135                 140
   Gly Asp Ile Asp Leu Thr Ser Xaa Ser Tyr Thr Met Ile
   145                 150                 155
<210> 2
   <211> 807
   <212> DNA
   <213> Artificial Sequence
<220>
   <223> Séquence partielle du vecteur pCARGHO
<220>
   <221> CDS
   <222> (94)..(690)
<400> 2
   gatcccgcga aattaatacg actcactata gggagaccac aacggtttcc ctctagaaat       60
   aattttgttt aactttaaga aggagatata cat atg agc gca acc ggt gca tat      114
                                         Met Ser Ala Thr Gly Ala Tyr
                                         1               5
   ccg gca acc ggt ccg tat ggt gca ccg gca ggt ccg ctg att gtt ccg      162
   Pro Ala Thr Gly Pro Tyr Gly Ala Pro Ala Gly Pro Leu Ile Val Pro
           10                  15                  20
   tat aat ctg ccg ctg cct ggt ggt gtt gtt ccg aaa atg ctg att acc      210
   Tyr Asn Leu Pro Leu Pro Gly Gly Val Val Pro Lys Met Leu Ile Thr
       25                  30                  35
   att ctg ggc acc gtt aaa ccg aat gca aat cgt att gca ctg gat ttt      258
   Ile Leu Gly Thr Val Lys Pro Asn Ala Asn Arg Ile Ala Leu Asp Phe
   40                  45                  50                  55
   cag cgt ggt aat gat gtg gcc ttt cat ttt aat ccg cgt ttc aat gaa      306
   Gln Arg Gly Asn Asp Val Ala Phe His Phe Asn Pro Arg Phe Asn Glu
                   60                  65                  70
   aac aac cgt cgt gtt att gtg tgc aat acc aaa ctg gat aac aat tgg      354
   Asn Asn Arg Arg Val Ile Val Cys Asn Thr Lys Leu Asp Asn Asn Trp
               75                  80                  85
   ggt cgt gaa gaa cgt cag agc gtt ttt ccg ttt gaa agc ggt aaa ccg        402
   Gly Arg Glu Glu Arg Gln Ser Val Phe Pro Phe Glu Ser Gly Lys Pro
           90                  95                  100
   ttt aaa atc cag gtt ctg gtt gaa ccg gat cat ttt aaa gtt gca gtt        450
   Phe Lys Ile Gln Val Leu Val Glu Pro Asp His Phe Lys Val Ala Val
       105                 110                 115
   aat gat gca cat ctg ctg cag tat aat cac cgt gtg aaa aaa ctg aac        498
   Asn Asp Ala His Leu Leu Gln Tyr Asn His Arg Val Lys Lys Leu Asn
   120                 125                 130                 135
   gag att agc aaa ctg ggt atc agc ggt gat att gat ctg acc agc acc        546
   Glu Ile Ser Lys Leu Gly Ile Ser Gly Asp Ile Asp Leu Thr Ser Thr
                   140                 145                 150
   agc tat acc atg att agc agc ggt gtt gat ctg ggt aca gaa aat ctg        594
   Ser Tyr Thr Met Ile Ser Ser Gly Val Asp Leu Gly Thr Glu Asn Leu
               155                 160                 165
   tat ttt cag agc gcc atg gat atc aat tcg gat ccg aat tcg agc tcc        642
   Tyr Phe Gln Ser Ala Met Asp Ile Asn Ser Asp Pro Asn Ser Ser Ser
           170                 175                 180
   gtc gac aag ctt gcg gcc gca ctc gag cac cac cac cac cac cac tga        690
   Val Asp Lys Leu Ala Ala Ala Leu Glu His His His His His His
       185                 190                 195
   gatccggctg ctaacaaagc ccgaaaggaa gctgagttgg ctgctgccac cgctgagcaa      750
   taactagcat aaccccttgg ggcctctaaa cgggtcttga ggggtttttt gctgaaa         807
<210> 3
   <211> 198
   <212> PRT
   <213> Artificial Sequence
<220>
   <223> Synthetic Construct
<400> 3
   Met Ser Ala Thr Gly Ala Tyr Pro Ala Thr Gly Pro Tyr Gly Ala Pro
   1               5                   10                  15
   Ala Gly Pro Leu Ile Val Pro Tyr Asn Leu Pro Leu Pro Gly Gly Val
               20                  25                  30
   Val Pro Lys Met Leu Ile Thr Ile Leu Gly Thr Val Lys Pro Asn Ala
           35                  40                  45
   Asn Arg Ile Ala Leu Asp Phe Gln Arg Gly Asn Asp Val Ala Phe His
       50                  55                  60
   Phe Asn Pro Arg Phe Asn Glu Asn Asn Arg Arg Val Ile Val Cys Asn
   65                  70                  75                  80
   Thr Lys Leu Asp Asn Asn Trp Gly Arg Glu Glu Arg Gln Ser Val Phe
                   85                  90                  95
   Pro Phe Glu Ser Gly Lys Pro Phe Lys Ile Gln Val Leu Val Glu Pro
               100                 105                 110
   Asp His Phe Lys Val Ala Val Asn Asp Ala His Leu Leu Gln Tyr Asn
           115                 120                 125
   His Arg Val Lys Lys Leu Asn Glu Ile Ser Lys Leu Gly Ile Ser Gly
       130                 135                 140
   Asp Ile Asp Leu Thr Ser Thr Ser Tyr Thr Met Ile Ser Ser Gly Val
   145                 150                 155                 160
   Asp Leu Gly Thr Glu Asn Leu Tyr Phe Gln Ser Ala Met Asp Ile Asn
                   165                 170                 175
   Ser Asp Pro Asn Ser Ser Ser Val Asp Lys Leu Ala Ala Ala Leu Glu
               180                 185                 190
   His His His His His His
           195
<210> 4
   <211> 250
   <212> PRT
   <213> Homo sapiens
<400> 4
   Met Ala Asp Asn Phe Ser Leu His Asp Ala Leu Ser Gly Ser Gly Asn
   1               5                   10                  15
   Pro Asn Pro Gln Gly Trp Pro Gly Ala Trp Gly Asn Gln Pro Ala Gly
               20                  25                  30
   Ala Gly Gly Tyr Pro Gly Ala Ser Tyr Pro Gly Ala Tyr Pro Gly Gln
           35                  40                  45
   Ala Pro Pro Gly Ala Tyr Pro Gly Gln Ala Pro Pro Gly Ala Tyr Pro
       50                  55                  60
   Gly Ala Pro Gly Ala Tyr Pro Gly Ala Pro Ala Pro Gly Val Tyr Pro
   65                  70                  75                  80
   Gly Pro Pro Ser Gly Pro Gly Ala Tyr Pro Ser Ser Gly Gln Pro Ser
                   85                  90                  95
   Ala Thr Gly Ala Tyr Pro Ala Thr Gly Pro Tyr Gly Ala Pro Ala Gly
               100                 105                 110
   Pro Leu Ile Val Pro Tyr Asn Leu Pro Leu Pro Gly Gly Val Val Pro
           115                 120                 125
   Arg Met Leu Ile Thr Ile Leu Gly Thr Val Lys Pro Asn Ala Asn Arg
       130                 135                 140
   Ile Ala Leu Asp Phe Gln Arg Gly Asn Asp Val Ala Phe His Phe Asn
   145                 150                 155                 160
   Pro Arg Phe Asn Glu Asn Asn Arg Arg Val Ile Val Cys Asn Thr Lys
                   165                 170                 175
   Leu Asp Asn Asn Trp Gly Arg Glu Glu Arg Gln Ser Val Phe Pro Phe
               180                 185                 190
   Glu Ser Gly Lys Pro Phe Lys Ile Gln Val Leu Val Glu Pro Asp His
           195                 200                 205
   Phe Lys Val Ala Val Asn Asp Ala His Leu Leu Gln Tyr Asn His Arg
       210                 215                 220
   Val Lys Lys Leu Asn Glu Ile Ser Lys Leu Gly Ile Ser Gly Asp Ile
   225                 230                 235                 240
   Asp Leu Thr Ser Thr Ser Tyr Thr Met Ile
                   245                 250
<210> 5
   <211> 5
   <212> PRT
   <213> Homo sapiens
<400> 5
   Gly Gly Val Val Pro
   1               5
<210> 6
   <211> 5
   <212> PRT
   <213> Homo sapiens
<400> 6
   Leu Ile Thr Ile Leu
   1               5
<210> 7
   <211> 155
   <212> PRT
   <213> Gorilla gorilla
<400> 7
   Ser Ala Pro Gly Ala Tyr Pro Ala Thr Gly Pro Tyr Gly Ala Pro Ala
   1               5                   10                  15
   Gly Pro Leu Ile Val Pro Tyr Asn Leu Pro Leu Pro Gly Gly Val Val
               20                  25                  30
   Pro Arg Met Leu Ile Thr Ile Leu Gly Thr Val Lys Pro Asn Ala Asn
           35                  40                  45
   Arg Ile Ala Leu Asp Phe Gln Arg Gly Asn Asp Val Ala Phe His Phe
       50                  55                  60
   Asn Pro Arg Phe Asn Glu Asn Asn Arg Arg Val Ile Val Cys Asn Thr
   65                  70                  75                  80
   Lys Leu Asp Asn Asn Trp Gly Arg Glu Glu Arg Gln Ser Val Phe Pro
                   85                  90                  95
   Phe Glu Ser Gly Lys Pro Phe Lys Ile Gln Val Leu Val Glu Pro Asp
               100                 105                 110
   His Phe Lys Val Ala Val Asn Asp Ala His Leu Leu Gln Tyr Asn His
           115                 120                 125
   Arg Val Lys Lys Leu Asn Glu Ile Ser Lys Leu Gly Ile Ser Gly Asp
       130                 135                 140
   Ile Asp Leu Thr Ser Ala Ser Tyr Asn Met Ile
   145                 150                 155
<210> 8
   <211> 155
   <212> PRT
   <213> Felis catus
<400> 8
   Ser Ala Pro Gly Ala Tyr Pro Ala Ala Gly Pro Phe Gly Val Pro Ala
   1               5                   10                  15
   Gly Pro Leu Thr Val Pro Tyr Asp Leu Pro Leu Pro Gly Gly Val Met
               20                  25                  30
   Pro Arg Met Leu Ile Thr Ile Leu Gly Thr Val Lys Pro Asn Ala Asn
           35                  40                  45
   Arg Leu Ala Leu Asp Phe Lys Arg Gly Asn Asp Val Ala Phe His Phe
       50                  55                  60
   Asn Pro Arg Phe Asn Glu Asp Asn Lys Arg Val Ile Val Cys Asn Thr
   65                  70                  75                  80
   Lys Leu Glu Asn Leu Trp Gly Lys Glu Glu Arg Gln Ser Thr Phe Pro
                   85                  90                  95
   Phe Glu Ser Gly Lys Pro Phe Lys Ile Gln Val Leu Val Glu Ser Asp
               100                 105                 110
   His Phe Lys Val Ala Val Asn Asp Ala His Leu Leu Gln Tyr Asn His
           115                 120                 125
   Arg Met Lys Asn Leu His Glu Ile Ser Lys Leu Gly Ile Ser Gly Asp
       130                 135                 140
   Ile Asp Leu Thr Ser Ala Ser His Thr Met Ile
   145                 150                 155
<210> 9
   <211> 155
   <212> PRT
   <213> Bos taurus
<400> 9
   Ser Ala Pro Gly Ala Tyr Pro Ala Ala Gly Pro Tyr Gly Ile Pro Ser
   1               5                   10                  15
   Gly Pro Leu Asn Val Pro Tyr Asp Leu Pro Phe Pro Gly Gly Ile Arg
               20                  25                  30
   Pro Arg Met Leu Ile Thr Ile Leu Gly Thr Val Lys Pro Asn Ala Asn
           35                  40                  45
   Arg Leu Ala Leu Asp Phe Lys Arg Gly Asn Asp Val Ala Phe His Phe
       50                  55                  60
   Asn Pro Arg Phe Asn Glu Asp Asn Arg Arg Val Ile Val Cys Asn Ser
   65                  70                  75                  80
   Lys Leu Asn Asn Asn Trp Gly Lys Glu Glu Arg Gln Met Val Phe Pro
                   85                  90                  95
   Phe Glu Ser Gly Lys Pro Phe Lys Ile Gln Val Leu Val Glu Pro Asp
               100                 105                 110
   His Phe Lys Val Ala Val Asn Asp Ala His Leu Leu Gln Tyr Asn His
           115                 120                 125
   Arg Val Lys Asn Phe Gly Glu Ile Ser Thr Leu Gly Ile Ser Gly Asp
       130                 135                 140
   Ile Thr Leu Thr Ser Ala Ser His Thr Met Ile
   145                 150                 155
<210> 10
   <211> 155
   <212> PRT
   <213> Mus musculus
<400> 10
   Ser Ala Pro Gly Gly Tyr Pro Ala Ala Gly Pro Tyr Gly Val Pro Ala
   1               5                   10                  15
   Gly Pro Leu Thr Val Pro Tyr Asp Leu Pro Leu Pro Gly Gly Val Met
               20                  25                  30
   Pro Arg Met Leu Ile Thr Ile Met Gly Thr Val Lys Pro Asn Ala Asn
           35                  40                  45
   Arg Ile Val Leu Asp Phe Arg Arg Gly Asn Asp Val Ala Phe His Phe
       50                  55                  60
   Asn Pro Arg Phe Asn Glu Asn Asn Arg Arg Val Ile Val Cys Asn Thr
   65                  70                  75                  80
   Lys Gln Asp Asn Asn Trp Gly Lys Glu Glu Arg Gln Ser Ala Phe Pro
                   85                  90                  95
   Phe Glu Ser Gly Lys Pro Phe Lys Ile Gln Val Leu Val Glu Ala Asp
               100                 105                 110
   His Phe Lys Val Ala Val Asn Asp Ala His Leu Leu Gln Tyr Asn His
           115                 120                 125
   Arg Met Lys Asn Leu Arg Glu Ile Ser Gln Leu Gly Ile Ser Gly Asp
       130                 135                 140
   Ile Thr Leu Thr Ser Ala Asn His Ala Met Ile
   145                 150                 155
<210> 11
   <211> 155
   <212> PRT
   <213> Rattus norvegicus
<400> 11
   Ser Ala Pro Gly Ala Tyr Pro Ala Thr Gly Pro Phe Gly Ala Pro Thr
   1               5                   10                  15
   Gly Pro Leu Thr Val Pro Tyr Asp Met Pro Leu Pro Gly Gly Val Met
               20                  25                  30
   Pro Arg Met Leu Ile Thr Ile Ile Gly Thr Val Lys Pro Asn Ala Asn
           35                  40                  45
   Ser Ile Thr Leu Asn Phe Lys Arg Gly Asn Asp Ile Ala Phe His Phe
       50                  55                  60
   Asn Pro Arg Phe Asn Glu Asn Asn Arg Arg Val Ile Val Cys Asn Thr
   65                  70                  75                  80
   Lys Gln Asp Asn Asn Trp Gly Arg Glu Glu Arg Gln Ser Ala Phe Pro
                   85                  90                  95
   Phe Glu Ser Gly Lys Pro Phe Lys Ile Gln Val Leu Val Glu Ala Asp
               100                 105                 110
   His Phe Lys Val Ala Val Asn Asp Val His Leu Leu Gln Tyr Asn His
           115                 120                 125
   Arg Met Lys Asn Leu Arg Glu Ile Ser Gln Leu Gly Ile Ile Gly Asp
       130                 135                 140
   Ile Thr Leu Thr Ser Ala Ser His Ala Met Ile
   145                 150                 155

## Description

### Domaine technique

La présente invention se rapporte à un nouveau procédé de purification en une seule étape de protéines recombinantes d'intérêt par affinité basée sur l'activité lectinique de tout ou partie du domaine lectinique, de préférence le domaine CRD (Carbohydrate Recognition Domain), d'une galectine.

La présente invention trouve ses applications principalement dans les laboratoires de recherche publics et privés, ainsi que dans l'industrie pharmaceutique ayant la nécessité de produire des protéines recombinantes dans le but d'études fondamentales ou d'intérêt thérapeutique.

Dans la description ci-dessous, les références entre crochets (**[ ]**) renvoient à la liste de références présentée à la fin du texte.

### État de la technique

Afin de produire à faible coût des protéines d'intérêt pures qui s'avèrent indispensables dans de nombreuses applications des sciences de la vie, il est essentiel de produire des quantités importantes de protéines recombinantes d'intérêt dans un hôte et de simplifier les étapes de traitement en aval.

Il existe de nombreuses méthodes de purification de protéines recombinantes d'intérêt de manière native (sans partenaire de fusion, i.e. tag), mais ce type de purification demeure complexe pour un rendement faible et une pureté parfois incomplète.

Aussi, des méthodes d'expression et de purification de protéines recombinantes fusionnées à un tag ont été proposées. Parmi les tags couramment utilisés figurent le tag histidine (composé d'au moins six histidines), la *maltose binding protein* (MBP) et la *glutathione S-transférase* (GST), les deux dernières étant des protéines.

Le tag histidine est utilisé dans le cadre de la technologie IMAC (Immobilized Metal Affinity). Cette méthode consiste à purifier une protéine recombinante fusionnée au tag histidine via l'interaction des noyaux imidazole avec des ions métalliques divalents (principalement le nickel) immobilisés sur une résine. La protéine de fusion est ensuite éluée avec une solution d'imidazole. Toutefois, du fait de sa faible spécificité, cette méthode de purification entraine de manière systématique une co-purification de contaminants et nécessite par la suite des étapes complémentaires. En outre, d'une part, le nickel est allergisant, fœtotoxique et néfaste pour l'environnement, et d'autre part, l'imidazole est également un composé fœtotoxique et reprotoxique. Ainsi, la présence possible de traces de nickel et d'imidazole dans les solutions de protéines recombinantes purifiées par IMAC en limite l'utilisation pour les applications *in vitro* / *in vivo.* De plus, les procédés à base d'ions métalliques peuvent conduire à des modifications de certains résidus des protéines ainsi purifiées [1, 2].

Quant à la MBP et la GST, elles sont utilisées pour purifier des protéines recombinantes par affinité pour l'amylose ou le glutathion respectivement. Du fait de l'encombrement stérique dû à la masse moléculaire de MBP, les protéines de fusion sont difficiles à cliver ce qui peut diminuer le rendement de production final de la protéine recombinante purifiée. La GST présente quant à elle, une faible affinité pour la résine de glutathion Sepharose [3, 4].

D'autres méthodes d'expression de protéines d'intérêt permettant une purification facile et efficace par chromatographie d'affinité sur des résines à faible coût, basée sur l'utilisation de lectines comme tag, ont été développées. L'avantage des lectines est qu'elles se lient spécifiquement et de façon réversible à certains polysaccharides. En effet il existe plusieurs classes de lectines différant par leur séquence d'acides aminés, leur structure tridimensionnelle mais également par la nature du site de fixation aux sucres. Ainsi des méthodes d'expression et de purification de protéines d'intérêt ont été développées à partir de lectines telles que la lectine LSL de champignon [5] ou la discoïdine provenant d'une amibe [6] sur des colonnes de chromatographie constituées de Sepharose 4B. Toutefois, dans ces méthodes, l'utilisation d'une résine non greffée entraine un risque de fixation non spécifique sur la résine et de ce fait une contamination par des protéines bactériennes.

On connait également une méthode de purification de protéines d'intérêt utilisant une résine greffée avec du mannose reconnue par la lectine LecB de P. *aeruginosa* [7]. Toutefois le mannose est un sucre dont la synthèse est complexe et onéreuse.

On connait aussi une méthode de purification de protéines d'intérêt en deux étapes utilisant une protéine de fusion comprenant le domaine lectinique d'une lectine hépatique de rat et une molécule d'intérêt, et avec un site de clivage par protéase inséré entre les deux [13]. Elle présente le désavantage de travailler en présence de chélateurs de cations, pouvant inhiber l'activité biologique de protéines d'intérêt.

Il existe un besoin de méthode d'expression et de purification de protéines d'intérêt palliant les inconvénients des procédés de l'art antérieur.

### Description de l'invention

La solution proposée par les Inventeurs pour exprimer des protéines auparavant insolubles (produites sous forme de corps d'inclusion) en améliorant leur solubilité, et purifier des protéines d'intérêt par affinité, présente l'avantage de ne pas modifier et/ou nuire à l'activité de la majorité des protéines.

Les inventeurs ont ainsi mis au point un nouveau procédé permettant de purifier des protéines recombinantes en une seule étape, avec une haute spécificité et un haut rendement. Ledit procédé de l'invention utilise le domaine lectinique d'une galectine ou partie dudit domaine conservant la capacité de lier le lactose, par exemple le domaine CRD_{SAT} d'une galectine, par exemple de la galectine-3 humaine, en tant que partenaire de fusion de la protéine d'intérêt. Ce tag lectinique permet ainsi la purification de la protéine d'intérêt en une seule étape de chromatographie d'affinité utilisant une résine de Sepharose greffée avec des molécules de lactose.

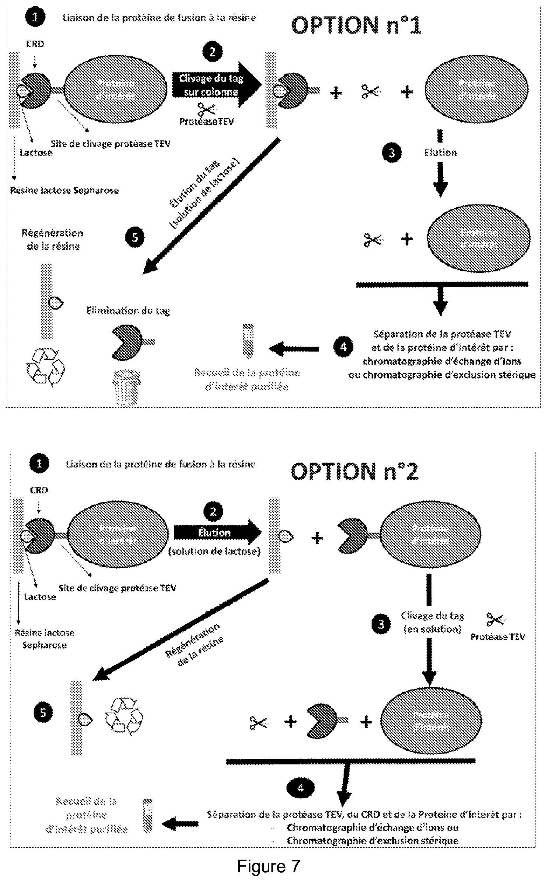

La présente invention a donc pour objet une protéine de fusion comprenant tout ou partie du domaine lectinique d'une galectine fusionnée avec une protéine d'intérêt, via une séquence comprenant un site de clivage par la protéase TEV (deux options de clivage possibles voir Fig. 7) où le domaine lectinique est le domaine CDRₛₐₜ de séquence SEQ ID NO : 1.

Par « domaine lectinique ou domaine GLECT » au sens de la présente invention, on entend le domaine des galectines liant les dérivés β-galactosyl comme par exemple le lactose et ses dérivés, et dont l'activité ne dépend pas de cations divalents. Le numéro d'accès dans la banque de données Conserved Domain du NCBI, du domaine consensuel GLECT, est cd00070.

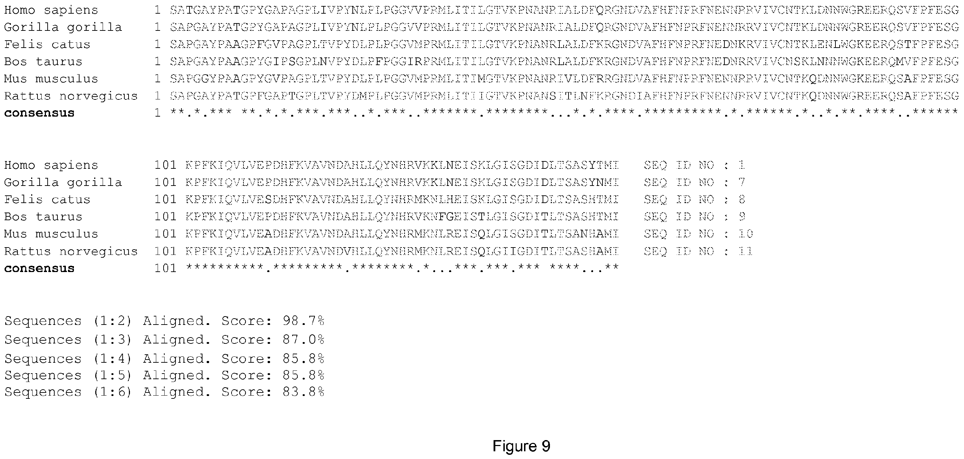

Le domaine CRD_{SAT} est un domaine extrêmement conservé (entre environ 83 et 99% d'homologie de séquence chez les mammifères) (cf. Fig. 9). Il fait partie de la superfamille de domaine GLECT (Galactose binding LECTin) capable de lier spécifiquement les β-galactosides tels que le lactose et ses dérivés.

La présente invention a également pour objet un vecteur d'expression d'une protéine de fusion, ledit vecteur comprenant les éléments suivants liés de manière fonctionnelle :
a) un promoteur placé en 5' des éléments b), c) et d) ;
b) une séquence codant pour le domaine CRDsAT de séquence SEQ ID NO : 1 ;
c) un site de clonage recevant une séquence codant pour une protéine d'intérêt ;
d) des signaux de terminaison de la transcription ;et dans lequel un site de clivage par protéase est localisé entre la séquence b) et le site de clonage c).

Par « promoteur » au sens de la présente invention, on entend une séquence ADN agissant en cis localisée en 5' du site d'initiation de la transcription de la séquence codant pour un polypeptide à laquelle une séquence ADN d'une ARN polymérase peut se lier et initier une transcription correcte, et comprenant éventuellement des activateurs.

Dans le vecteur d'expression de la présente invention, la séquence correspondant à tout ou partie du domaine lectinique d'une galectine est par exemple localisée à proximité, en amont du site de clonage, et en aval du promoteur. Une alternative est de placer ladite séquence à proximité, après le site de clonage. Dans les deux cas, la fusion de ladite séquence avec celle de la molécule d'intérêt permet la purification sur résine. La séquence b) code pour une partie du domaine lectinique liant le lactose, le domaine CRD_{SAT} de séquence SEQ ID NO : 1.

Selon un mode de réalisation particulier de la présente invention, le site de clivage par protéase est reconnu par la protéase TEV.

Selon un mode de réalisation particulier de la présente invention, le vecteur d'expression est le vecteur d'expression pCARGHO (pour **CA**rbohydrate **R**ecognition domain of **G**alectine 3 from **HO**mo sapiens) dérivé du plasmide pET.

La présente invention a également pour objet un procédé de production d'une protéine d'intérêt purifiée, ledit procédé comprenant :
a) la préparation d'un vecteur d'expression selon la présente invention;
b) la transformation d'une cellule hôte avec ledit vecteur d'expression;
c) la culture de ladite cellule hôte ainsi transformée dans des conditions permettant l'expression de la protéine de fusion et favorisant sa solubilisation dans les cellules hôtes ; et
d) la purification de la molécule d'intérêt.

Dans le procédé de la présente invention, l'étape de purification d) est réalisée par exemple par liaison de la protéine de fusion via le domaine lectinique de la galectine ou partie dudit domaine à un support de chromatographie greffé avec des molécules de lactose, de préférence à une résine d'agarose ou de Sepharose greffée avec des molécules de lactose, puis (i) clivage par protéase pour retirer le domaine lectinique de la galectine ou partie dudit domaine (i.e. séquence tag), élution et séparation de la molécule d'intérêt et de la protéase, ou (ii) élution de la protéine de fusion, clivage par protéase en solution, et séparation de la protéine d'intérêt et de la protéase.

Selon un mode de réalisation particulier de la présente invention, l'étape de séparation de la protéase et de la protéine d'intérêt est réalisée par chromatographie d'échanges d'ions ou chromatographie d'exclusion stérique ou chromatographie d'interactions hydrophobes.

La présente invention a également pour objet un procédé de purification d'une molécule d'intérêt, ledit procédé comprenant :
Option n°1 :
   a) la liaison de la protéine de fusion de la présente invention sur un support de chromatographie greffé avec des molécules de lactose, de préférence une colonne de résine d'agarose ou de Sepharose greffée avec des molécules de lactose ;
   b) le clivage par protéase de la protéine de fusion au niveau du site de clivage spécifique ;
   c) l'élution de la molécule d'intérêt purifiée ;
   d) la séparation de la protéase, de la protéine d'intérêt ainsi purifiée.
      Selon un mode de réalisation particulier de la présente invention, l'étape d) est réalisée par chromatographie d'échanges d'ions ou chromatographie d'exclusion stérique ou chromatographie d'interactions hydrophobes.
      Le domaine lectinique ou partie dudit domaine (i.e. le tag) est éliminé de la colonne par élution par compétition avec une solution de lactose, et la colonne est régénérée.
Option n°2 :
   a) la liaison de la protéine de fusion de la présente invention sur un support de chromatographie greffé avec des molécules de lactose, de préférence une colonne de résine d'agarose ou de Sepharose greffée avec des molécules de lactose ;
   b) l'élution de la protéine de fusion par compétition avec une solution de lactose ;
   c) le clivage par protéase de la protéine de fusion au niveau du site spécifique, en solution ;
   d) la séparation du domaine lectinique ou partie dudit domaine (*i.e.* le tag) et de la protéase TEV, de la molécule d'intérêt ainsi purifiée.

Selon un mode de réalisation particulier de la présente invention, l'étape d) est réalisée par chromatographie d'échanges d'ions ou chromatographie d'exclusion stérique.

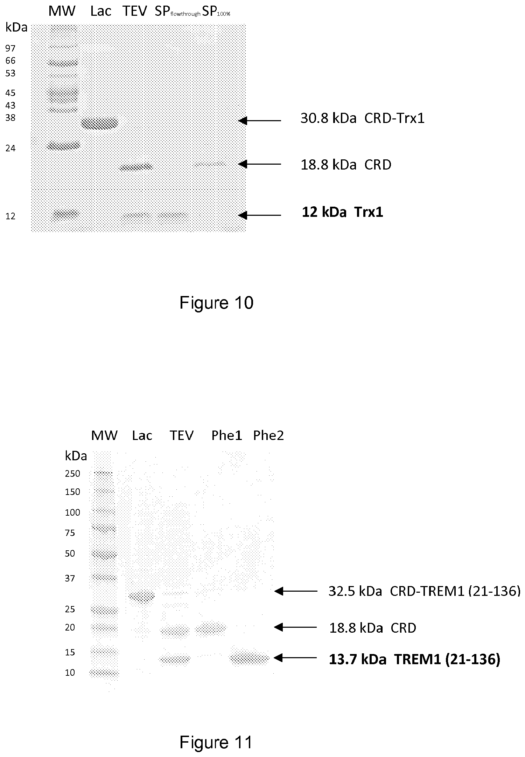

Le procédé de l'invention présente l'avantage de n'utiliser aucun composé toxique, cancérogène ou tératogène. En outre, l'activité lectinique du domaine CRD de la galectine-3 est bien plus spécifique que toutes les autres méthodes de purification et ne nécessite qu'une seule étape pour obtenir un degré de pureté supérieur à 95%. Par ailleurs les conditions d'élution de la protéine de fusion sont optimales pour que le clivage du tag s'effectue par la protéase TEV. Les agents réducteurs sont acceptés sans limite. Le tag CRD, en particulier CRD_{SAT}, est de très faible poids moléculaire (17,3 kDa, 18,8kDa pour la forme obtenue après clivage par la protéase TEV), ce qui permet son élimination facile par chromatographie d'exclusion de taille. En outre il est fortement structuré en feuillets β ; ce qui lui confère une très grande stabilité. Enfin son point isoélectrique est très basique (pl=9,3, pl=8,7 pour la forme obtenue après clivage par la protéase TEV), ce qui permet sa capture facile sur une colonne échangeuse de cations. Il est possible de conjointement éliminer le CRD et la protéase TEV dont le point isoélectrique est également basique (pl=8,8) en les fixant sur colonne échangeuse de cations. La protéine d'intérêt est alors éluée pure dans la fraction non retenue par la résine (figure 10).

La présente invention a également pour objet le partenaire de fusion CRD_{SAT} (ou tag) dérivé d'une galectine. La séquence en acides aminés du domaine CRD_{SAT} est la séquence SEQ ID NO : 1, dans laquelle l'acide aminé en position 36 peut être une arginine ou une lysine, tout préférentiellement une lysine, et/ou dans laquelle l'acide aminé en position 152 peut être une alanine ou une thréonine, tout préférentiellement une thréonine. Le partenaire de fusion CRD présente également la propriété de solubiliser des protéines décrites comme insolubles, auparavant obtenues après renaturation longue et fastidieuse de corps d'inclusion en urée ou chlorure de guanidinium. Par exemple, le récepteur membranaire humain TREM-1 et plus particulièrement son domaine extracellulaire [14-15].

### Brève description des figures

- La figure 1 représente un schéma circulaire et linéaire du vecteur pCARGHO.
- La figure 2 représente les principales caractéristiques du vecteur pCARGHO (SEQ ID NOs : 2 et 3).
- La figure 3 représente une structure schématique de la galectine-3 humaine.
- La figure 4 représente la séquence protéique de la galectine-3 humaine et des 3 CRD conçus (SEQ ID NO : 4).
- La figure 5 représente la structure 3D du domaine lectinique CRD de la galectine-3 en interaction avec le lactose.
- La figure 6 représente le suivi de purification de la galectine-3 humaine par chromatographie d'affinité pour le lactose.
- La figure 7 représente le schéma de la méthode de purification de la présente invention par affinité pour le lactose.

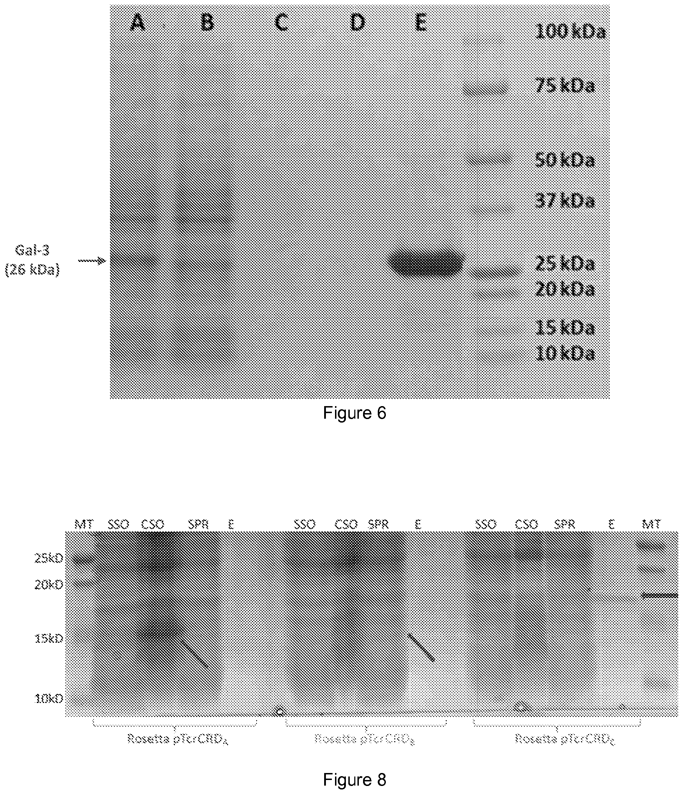

- La figure 8 représente le suivi de purification de 3 formes de galectine-3 humaine tronquée par chromatographie d'affinité pour le lactose. Les CRD_{LITIL} (A), CRD_{GGVVP} (B) et CRD_{SAT} (C) ont été exprimés dans des bactéries *E. coli* Rosetta2 (DE3). Les bactéries ont été lysées et des tests de purification ont été effectués sur colonne de lactose-agarose. À chaque étape de purification, un échantillon a été prélevé pour être déposé sur gel d'acrylamide (SDS-PAGE). Après migration électrophorétique, le gel a été coloré au bleu de Coomassie. **SSO :** surnageant d'extrait bactérien avant passage sur colonne d'affinité lactose. **CSO :** culot d'extrait bactérien avant passage sur colonne d'affinité lactose. **SPR** : surnageant après le passage sur la colonne d'affinité lactose. **E** : fraction d'élution. **<---**: emplacement de la protéine d'intérêt.
- La figure 9 représente un alignement de séquences en acides aminés du domaine CRD_{SAT} de la galectine-3 chez différents mammifères, ainsi que la séquence consensus dudit domaine CRD_{SAT}.
- La figure 10 représente le suivi de purification de la thiorédoxine bactérienne (Trx1) par chromatographie d'affinité pour le lactose selon le procédé de l'invention.
- La figure 11 représente le suivi de purification de TREM-1 domaine extracellulaire résidus 21 à 136 par chromatographie d'affinité pour le lactose selon le procédé de l'invention.

### EXEMPLES

### EXEMPLE 1 : RECHERCHE DE LA FORME OPTIMALE DE GALECTINE-3 HUMAINE TRONQUÉE : SUIVI DE PURIFICATION DE 3 FORMES TRONQUÉES PAR CHROMATOGRAPHIE D'AFFINITÉ POUR LE LACTOSE.

### La galectine-3

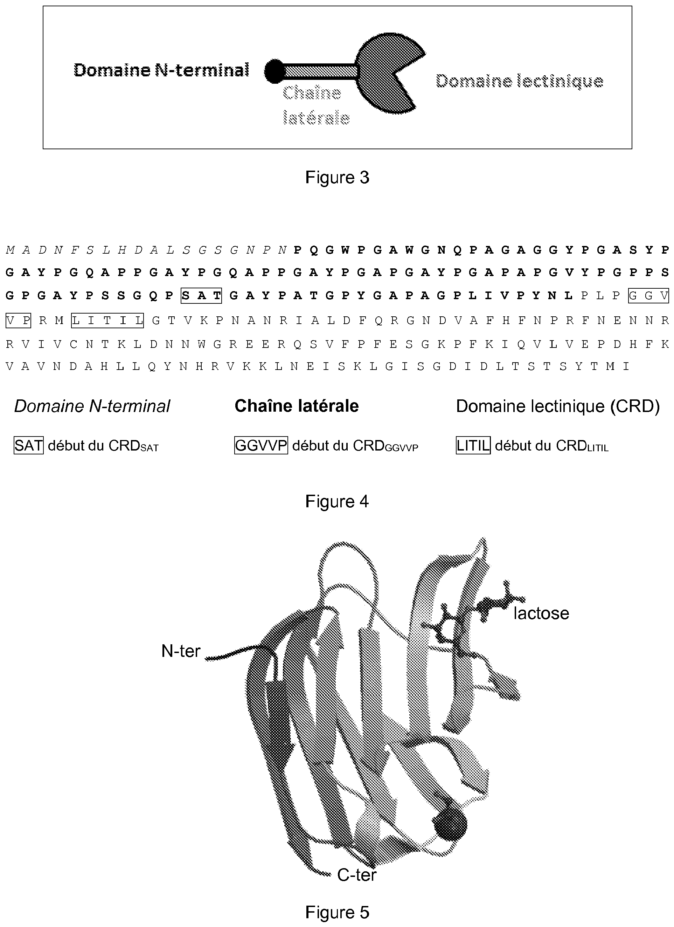

La galectine 3 est une lectine animale de 243 à 286 acides aminés, selon l'espèce (Cooper, Biochim. & Biophys. Acta, 1572(2-3): 209-231, 2002) [8]. D'environ 30 kDa, elle est composée d'un petit domaine N-terminal, d'une chaîne latérale et d'un domaine lectinique C-terminal (CRD : carbohydrate Recognition Domain) (figures 3 et 4) (Leffler et al., Glycoconj. J., 19 : 433-440, 2004 ; Ochieng et al., Biochim. & Biophys; Acta, 1379: 97-106, 1998) [9, 10]. Formé de plusieurs feuillets bêta (Salomonsson et al., J. Biol. Chem., 285 : 35079-35091, 2010; Seetharaman et al., J. Biol. Chem., 273 : 13047-13052, 1998) [11, 12], le domaine lectinique permet à la protéine d'interagir avec des molécules contenant des résidus β-galactosides, par exemple le lactose (figure 5).

Compte tenu de ses propriétés lectiniques, la galectine-3 a pu être purifiée en une seule étape et de façon spécifique par chromatographie d'affinité, en utilisant une résine d'agarose greffée avec des molécules de lactose, selon le protocole décrit précédemment (figure 6).

Pour ce faire, la galectine-3 (forme entière) a été exprimée dans des bactéries *E. coli* C41(DE3) puis purifiée sur colonne de lactose-agarose. A chaque étape de purification, un échantillon a été prélevé pour être déposé sur gel d'acrylamide (SDS-PAGE). Après migration électrophorétique, le gel a été coloré au Bleu de Coomassie. **A** : extrait bactérien avant passage sur colonne d'affinité lactose. **B** : extrait bactérien après passage sur colonne d'affinité lactose. **C et D** : lavages de la colonne. **E** : fraction d'élution de la galectine-3 ; élution avec une solution de PBS + lactose 150 mM.

Les résultats sont présentés dans la figure 6.

La facilité de mise en œuvre de cette purification ainsi que l'important degré de pureté obtenu ont orienté les inventeurs vers l'idée de développer un partenaire de fusion (*tag,* en anglais) destiné à la purification de protéines recombinantes par chromatographie d'affinité pour le lactose. L'idée a été d'utiliser une partie du domaine lectinique de la galectine 3 (CRD) capable de lier le lactose pour constituer ce partenaire de fusion et permettre une purification en une seule étape au cours de laquelle ce partenaire pourra être clivé par la protéase TEV (figure 7).

### Recherche de la forme optimale de galectine-3 tronquée

### Création de la séquence nucléotidique optimisée codant pour le CRD_{SAT} et intégration dans un vecteur d'expression de type pET-20b

A partir de la séquence nucléotidique de la galectine-3 humaine, 3 séquences de CRD codant pour 3 protéines CRD différentes ont été clonées : CRD_{LITIL} (14 kDa), CRD_{GGVVP} (15 kDa) et CRD_{SAT} (∼17 kDa, forme naturelle, non synthétique, non optimisée) (figure 4).

Il en ressort que les 3 CRD sont exprimés mais ont une solubilité variable. Ainsi le CRD_{LITIL} (A) est sous forme totalement insoluble (dans le culot) et n'a pas été retrouvé dans l'éluat donc impossible à purifier. Le CRD_{GGVVP} (B) est produit en faible quantité en partie sous forme soluble mais a perdu sa fonction lectinique et n'a donc pas pu être purifié. Le CRD_{SAT} (C) est produit totalement sous forme soluble et a pu être purifié (retrouvé dans l'éluat) (figure 8).

Compte tenu des différents tests réalisés, le choix s'est porté sur le CRD_{SAT} pour constituer le partenaire de fusion souhaité. Les caractéristiques physicochimiques de cette protéine, déterminées *in silico,* sont les suivantes :
Nombre d'acides aminés: **156**
Poids moléculaire : **17 360.9 Da**
Point isoélectrique: **9.30**

Nombre total d'acides aminés de charge négative (Asp + Glu): 13 Nombre total d'acides aminés de charge positive (Arg + Lys): 17 Coefficient d'extinction molaire: **12 950** M⁻¹ cm⁻¹ (à 280 nm).
Abs 0.1% (=1 g/l) 0.746, sous réserve que toutes les cystéines soient sous forme réduite.

La séquence codant pour le CRD_{SAT} a été optimisée *in silico* afin de favoriser l'expression de cette protéine hétérologue dans *E. coli* (suppression des biais de codons) et de permettre d'accroitre sa solubilité (substitution de l'arginine 36 en lysine), ainsi que sa rigidité par augmentation de l'encombrement (substitution de l'arginine 152 en thréonine) permettant ainsi à la protéase de cliver 14 acides aminés en aval.

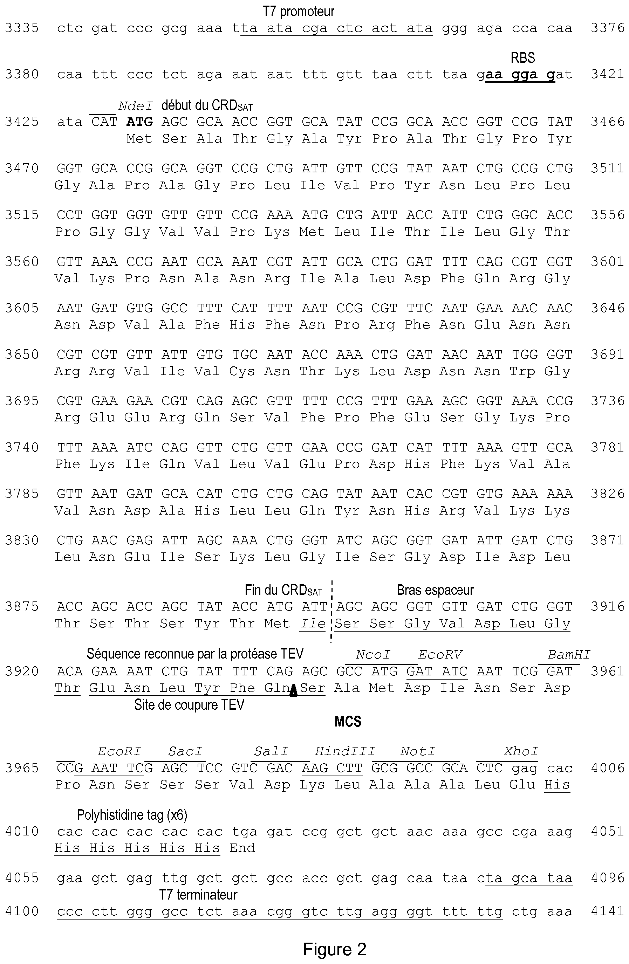

Cette séquence CRD_{SAT} optimisée a été intégrée dans un vecteur d'expression de type pET-20b : le vecteur pCARGHO (Figure 2), afin de permettre l'expression et la purification d'une protéine d'intérêt selon l'Exemple 3.

### EXEMPLE 2 : LE pCARGHO : MATÉRIEL ET MÉTHODES

Le plasmide pCARGHO permet la production d'une protéine de fusion constituée, dans l'ordre : du CRD_{SAT} de la galectine-3 humaine, d'un bras espaceur, permettant de la flexibilité, d'un site de clivage par la protéase TEV et de la protéine d'intérêt.

La souche d'*E*. *coli* utilisée doit obligatoirement être de type (DE3), c'est-à-dire posséder le gène de la T7 ARN polymérase intégré dans son génome.

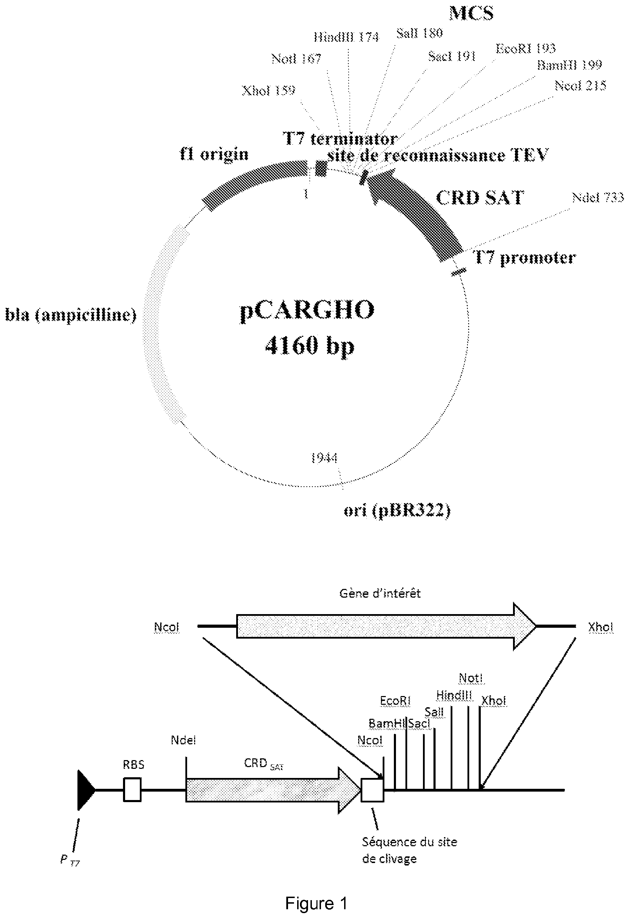

Le plasmide pCARGHO est dérivé du pET20b(+) de Novagen et comprend les éléments suivants (figure 1) :

| **ELEMENTS** | **POSITION** |
|---|---|
| Origine de réplication | 1944 |
| Promoteur T7 | 797-813 |
| Site de fixation au ribosome : RBS | 742-747 |
| Séquence CRD_{SATG} | 242-736 |
| Séquence site de reconnaissance TEV | 221-241 |
| Site de clonage multiple : MCS | 159-215 |
| Terminateur T7 | 26-72 |
| Origine F1 | 3694-4149 |
| Gène *bla* de résistance à l'ampicilline | 2705-3562 |

Les principales caractéristiques du vecteur pCARGHO sont indiquées dans la figure 2 (SEQ ID NOs : 2 et 3).

### Clonage dans le plasmide pCARGHO

Le protocole ci-dessous est un exemple de clonage d'un fragment PCR d'une protéine d'intérêt dans le vecteur pCARGHO. Pour certaines expériences (digestion enzymatique, réaction de ligature, transformation bactérienne), il convient de se référer notamment aux fournisseurs des réactifs utilisés.

Le fragment PCR utilisé doit contenir le site de restriction Ncol à son extrémité 5' et un autre site de restriction BamHI, EcoRI, Sacl, SalI, Hindlll, Notl et Xhol, à son extrémité 3'. Les extrémités peuvent être indifféremment franches ou prolongées par une adénosine en 3'. Il faut s'assurer que la composition du fragment PCR garantisse le respect du cadre de lecture à partir du codon d'initiation ATG.
1) Digérer 1 µg de plasmide pCARGHO et 1 µg de fragment PCR en parallèle, dans 20 µl de 1X TP de digestion (10x stock) avec 10 unités de Ncol et 10 unités de la deuxième endonucléase choisie (selon site disponible dans le MCS), à 37°C pendant 1h à 2h. Les enzymes seront ensuite inactivées à 65°C pendant 10 minutes.
2) Vérifier la digestion complète du plasmide après migration sur gel d'agarose (5 µl).
3) Purifier le plasmide et le fragment de PCR digérés, dans le but d'éliminer le fragment MCS du plasmide et les extrémités libérées du fragment de PCR. La purification peut être effectuée sur gel et/ou à l'aide de kits spécifiques.
4) Doser le plasmide et le fragment PCR (insert).
5) Préparer le mélange de ligature suivant :
   30-50 ng du plasmide digéré
   50 ng d'insert dans 1 µl de tampon de ligature stock 10x
   1 µl de T4 DNA ligase
   H₂O qsp 10 µl
6) Incuber de 10 minutes à 2h, à température ambiante ou à 16°C pendant 16h.
7) Transformer les bactéries de clonage compétentes (type TOP10, DH5a) : prélever 2 µl de la réaction de ligature et ajouter 50 µl de bactéries. Incuber 30 minutes dans la glace. Chauffer à 42°C pendant 1 minute.
8) Ajouter 200 µl de milieu SOC (ou LB) et incuber à 37°C de 20 minutes à 1h. Étaler sur gélose sélective (LB Agar, 100 µg/ml d'ampicilline). Incuber une nuit à 37°C.
9) Rechercher la présence de clones positifs (vérifier la présence de l'insert dans le plasmide) par des méthodes bien connues de l'art telles que PCR directement sur colonies, mini-préparation d'ADN plasmidique, digestion par les enzymes de restriction adaptées et migration sur gel d'agarose, test de surexpression de la protéine de fusion.
10) Faire séquencer les plasmides extraits des clones supposés positifs pour valider le clonage moléculaire.

### Expression de la protéine de fusion « CRD_{SAT} - protéine d'intérêt »

### Test d'expression de la protéine de fusion

1) Transformer des bactéries d'expression compétentes [type BL21 (DE3)] avec le vecteur pCARGHO-X (X étant la séquence codant pour la protéine d'intérêt fusionnée au tag CRD_{SAT})
2) Inoculer des colonies isolées sur gélose dans 5 ml de milieu LB + ampicilline 100 µg/ml et mettre en culture jusqu'à 2x10⁸ cellules/ml (A₆₀₀ = 0,5-0,6).
3) Diviser l'échantillon en deux cultures de 2,5 ml.
4) Ajouter l'IPTG dans l'une des cultures à une concentration finale de 1 mM. Incuber les deux cultures à 37°C pendant 2-3h.
5) Prélever 500 µl de chaque culture. Centrifuger à vitesse maximale pendant 1 minute, jeter les surnageants et suspendre les culots bactériens avec 100 µl de tampon de Laemmli 1X.
6) Faire bouillir les échantillons pendant 1 minute. Déposer 10 µl de chaque échantillon et un marqueur de poids moléculaire sur un gel SDS-PAGE au pourcentage adapté. Faire migrer et révéler par coloration au bleu de Coomassie par exemple.

### Production de la protéine de fusion

Une fois que le séquençage et les tests d'expression ont été validés, la production de la protéine d'intérêt fusionnée au tag CRD_{SAT} (CRD_{SAT} - protéine d'intérêt) pourra être effectuée selon la procédure ci-dessous.
1) Transformer des bactéries d'expression compétentes [type BL21 (DE3)] avec le vecteur pCARGHO-X (X étant la séquence codant pour la protéine d'intérêt fusionnée au tag CRD_{SAT}). Effectuer une pré-culture dans du milieu LB Luria Bertani + ampicilline 100 µg/ml, à 37°C pendant 16h.
2) Inoculer cette pré-culture au 100^{e} dans 1 litre de milieu riche de type LB Luria Bertani + ampicilline 100 µg/ml.
3) Mettre en culture à 37°C jusqu'à 2x10⁸ cellules/ml (A₆₀₀ = 0,5-0,6). Ajouter l'IPTG à une concentration finale de 1 mM. Incuber les bactéries à 37°C sous agitation pendant 2-3h.
4) prélever 20 µl de la suspension bactérienne brute (A), centrifuger (4000 g pendant 20 minutes) la culture, éliminer le surnageant et suspendre le culot bactérien dans 25 ml de tampon de lyse.
   *Différents adjuvants peuvent permettre d'éviter la protéolyse (Ethylène Diamine tétra-Acétique (EDTA), fluorore de phénylméthylsulfonyle (PMSF), etc...) ou l'oxydation (dithiothréitol (DTT), β-mercapto-éthanol).*
5) Placer l'échantillon dans la glace et lyser les bactéries à l'aide d'un sonicateur ou d'une French Press. Prélever 20 µl de cette suspension bactérienne lysée (A').
6) Centrifuger la suspension bactérienne lysée à 20 000 g pendant 20 minutes. récupérer le surnageant protéique (Sp). Prélever 20 µl de ce surnageant (B). Diluer si nécessaire avec du tampon de lyse ou du tampon colonne.

### Purification de la protéine de fusion « CRD_{SAT} - protéine d'intérêt »

### Purification en mode automatisé

1) Après filtration sur membrane 0,45 µm, injecter l'échantillon Sp sur une résine lactose-Sepharose® (15 à 20 ml) préalablement équilibrée avec 5 CV (volume colonne) de tampon colonne. Prélever 20 µl de la fraction soluble écoulée à travers la colonne de chromatographie (C).
2) Laver la colonne avec 10 CV de tampon colonne. Prélever 20 µl du liquide de lavage (D) en sortie de colonne.
3) Éluer la protéine de fusion avec 1-2 CV de tampon colonne + 150 mM de lactose. Collecter les fractions correspondantes (taille des fractions : environ 1/5^{e} du volume de la colonne). Prélever 20 µl d'éluat (E). Suivre la protéine de fusion présente dans les fractions recueillies, par absorption à 280 nm et/ou par méthodes colorimétriques (BCA, Bradford, etc...).
   *Au cas où le tag CRD_{SAT} serait indésirable pour les applications ultérieures de la protéine d'intérêt, il est possible de le cliver après l'élution de la protéine de fusion (cf. § clivage de la protéine de fusion).*
4) Rassembler les fractions contenant la protéine de fusion et concentrer si nécessaire. Conserver la protéine d'intérêt à -80°C après congélation dans l'azote liquide avec ou sans ajout de 30 à 50% de glycérol.
5) Evaluer la qualité de la purification par SDS-PAGE + coloration : diluer les échantillons A à E au demi dans du tampon Laemmli 2X puis porter à ébullition (95°C, 5 minutes) avant de les faire migrer sur un gel d'acrylamide au pourcentage approprié.
6) Régénérer la colonne avec 5 CV de solution NaCl 2M puis 5 CV d'eau. Conserver la colonne en eau + éthanol 20% ou en Tris-HCl 20 mM EDTA 1 mM.

### Purification en mode batch

1) Déposer l'échantillon Sp sur une résine lactose agarose (10-15 ml) préalablement équilibrée avec 5 à 10 CV de tampon colonne (Tris 20 mM, EDTA 5 mM, NaCl 150 mM, pH à adapter selon la protéine d'intérêt) et mettre sous agitation faible 1 à 2 heures. Disposer ensuite dans une colonne avec disque fritté. Prélever 20 µl de la fraction soluble écoulée à travers la colonne de chromatographie (C).
2) Laver la colonne avec 10 CV de tampon colonne puis avec 10 CV de tampon PBS. Prélever 20 µl en sortie de colonne.
3) Éluer la protéine de fusion avec 2,5 CV de tampon PBS + lactose 150 mM.
4) Évaluer la qualité de la purification, doser la protéine de fusion puis la stocker selon les procédures décrites dans le § purification en mode automatisé.
5) Régénérer la colonne avec 10 CV de solution NaCl 2 M.

### Clivage de la protéine de fusion « CRD_{SAT} - protéine d'intérêt »

Le clivage de la protéine de fusion s'effectue par la protéase TEV dont le site de coupure se situe en C-terminal du tag CRD_{SAT}. Intervenant après l'élution de la protéine de fusion, il doit être suivi d'une étape de chromatographie soit échangeuse de cations, soit d'exclusion stérique, pour séparer le tag CRD_{SAT} (pl=8.7, PM=18.8 kDa) et la protéase TEV (pl=8,8, PM=27 kDa) de la protéine d'intérêt.
1) Diluer la solution concentrée de protéine de fusion à une concentration de 1-2 mg/ml dans le tampon de clivage : 25 mM Tris-HCl, pH 8, 150-500 mM NaCl, 15 mM β-mercapto-éthanol. Prélever 20 µl d'échantillon (A).
2) Ajouter la protéase TEV à un ratio de 1:100, soit 1 mg (10 000 unités) de protéase pour 100 mg de protéine de fusion. Le ratio « idéal » peut faire l'objet d'une optimisation.
3) Incuber le mélange à 4°C pendant une nuit ou encore à température ambiante ou à 30-37°C pendant des temps plus courts, le facteur limitant étant la stabilité de la protéine d'intérêt. Prélever 20 µl d'échantillon après clivage (B).
4) Déposer les échantillons A et B sur gel SDS-PAGE pour vérifier l'efficacité du clivage.
5) Éliminer le tag CRD_{SAT} ainsi que la protéase TEV par :
   a) chromatographie échangeuse de cations :
      i. Dialyser ou diluer le mélange de réaction de clivage dans le but de faire baisser la force ionique avec un tampon 20 mM Tris-HCl, 25 mM NaCl, pH 7. La concentration saline ne doit pas excéder 25 mM.
      ii. Equilibrer la colonne SP-Sepharose® ou équivalent avec 5 CV de tampon colonne (selon les recommandations du fournisseur).
      iii. Déposer le mélange sur la colonne et collecter immédiatement la fraction non retenue qui contient la protéine d'intérêt (à la condition que la protéine d'intérêt ait un pl acide (<7)).
      iv. Concentrer la protéine d'intérêt si nécessaire et la conserver à 80°C après congélation dans l'azote liquide avec ou sans ajout de 30 à 50% de glycérol.
      v. Régénérer la colonne avec 5 CV tampon 2 M NaCl (élution du tag CRD_{SAT} et de la protéase TEV), 5 CV d'eau. Conserver en eau + éthanol 20%.
   b) chromatographie d'exclusion stérique
      i. Équilibrer la colonne Superdex 75® ou Superdex 200® ou équivalent avec 2 CV de tampon colonne (selon les recommandations du fournisseur).
      ii. Injecter le mélange sur la colonne et collecter les fractions correspondant à la protéine d'intérêt sans tag [en fonction de son poids moléculaire (taille)].
      iii. Concentrer la protéine d'intérêt si nécessaire et la conserver à 80°C après congélation dans l'azote liquide ou ajout de 30 à 50% de glycérol
         *Si le tag a un poids moléculaire (PM) très différent de celui de la protéine d'intérêt, une détection par coloration du gel est suffisante. Si en revanche son PM est très proche, une détection plus spécifique par Western blot est possible grâce à des anticorps anti-CRD_{SAT}.*
      iv. Conserver la colonne en eau + éthanol 20%.

*Addendum : Si la digestion par TEV n'est pas effectuée et au cas où le lactose serait gênant pour les applications ultérieures de la protéine purifiée, il convient de dialyser ou d'effectuer une colonne de gel filtration.*

### EXEMPLE 3: SUIVI DE PURIFICATION DE LA THIOREDOXINE BACTÉRIENNE (Trx1) PAR CHROMATOGRAPHIE D'AFFINITÉ POUR LE LACTOSE

La Trx1 fusionnée au CRD_{SAT} a été exprimée dans des bactéries *E. coli* Rosetta2 (DE3), selon le protocole précédemment décrit.

Les bactéries ont été lysées et la purification a été effectuée sur colonne de lactose-agarose, selon le protocole précédemment décrit.

A différentes étapes de purification, un échantillon a été prélevé pour être déposé sur gel d'acrylamide (SDS-PAGE).

Après migration électrophorétique, le gel a été coloré au bleu de Coomassie.

Les résultats sont présentés dans la figure 10.

**MW :** poids moléculaires.

**Lac:** fraction purifiée de protéine de fusion Trx1-CRD_{SAT} sur résine lactose-agarose, élution avec 150mM de lactose.

**TEV** : fraction après coupure avec la protéase TEV, au 1/100^{e}, sur la nuit à T°ambiante.

**SP flow through** : fraction non retenue après injection sur colonne SP Sepharose (échangeuse de cations) du milieu réactionnel de digestion (TEV).

**SP 100%** : fraction éluée avec 1M de NaCl (SP Sepharose).

### EXEMPLE 4 : SUIVI DE PURIFICATION DU RÉCEPTEUR MEMBRANAIRE HUMAIN TREM-1 (DOMAINE EXTRACELLULAIRE) PAR CHROMATOGRAPHIE D'AFFINITÉ POUR LE LACTOSE

TREM-1 (21-136) fusionné au CRD_{SAT} a été exprimée dans des bactéries *E. coli* C41 (DE3), selon le protocole précédemment décrit.

Les bactéries ont été lysées et la purification a été effectuée sur colonne de lactose-agarose, selon le protocole précédemment décrit.

A différentes étapes de purification, un échantillon a été prélevé pour être déposé sur gel d'acrylamide (SDS-PAGE).

Après migration électrophorétique, le gel a été coloré au bleu de Coomassie.

Les résultats sont présentés dans la figure 11.

**MW :** poids moléculaires.

**Lac :** fraction purifiée de protéine de fusion CRD-TREM-1(21-136) sur résine lactose-agarose, élution avec 150mM de lactose.

**TEV** : fraction après coupure avec la protéase TEV, au 1/100^{e}, sur la nuit à T°ambiante.

**Phe1:** fraction non retenue après injection sur colonne Phenyl Sepharose (interactions hydrophobes) du mélange réactionnel de digestion (TEV), contenant le tag CRD.

**Phe2** : fraction, éluée à 300 mM de sulfate d'ammonium, contenant TREM-1, 13,7 kDa, résidus 21 à 136 avec un rendement de 4 mg/L de culture bactérienne.

### Liste des références

1. Stadtman et al., Free Radic Biol Med., 9(4):315-25, 1990
2. Chen et al., Mol Cell Biol. May; 26(10): 3728-3737, 2006
3. Andberg et al., Protein Science, 16:1751-1761, 2007
4. Ortiz-Salmeron et al., Eur. J. Biochem., 268, 4307-4314, 2001
5. Demande internationale WO 2009121994
6. Demande internationale WO 9966053
7. Tielker et al., Biotechniques, 41(3) : 327-332, 2006
8. Cooper, Biochim. & Biophys. Acta, 1572(2-3) : 209-231, 2002
9. Leffler et al., Glycoconj. J., 19 : 433-440, 2004
10. Ochieng et al., Biochim. Biophys. Acta, 1379 : 97-106, 1998
11. Salomonsson et al., J. Biol. Chem., 285 : 35079-35091, 2010
12. Seetharaman et al., J. Biol. Chem., 273 : 13047-13052, 1998
13. Taylor et al., Biochem. J., 274 : 575-580, 1991
14. Kelker et al., J.Mol.Biol. 342, 1237-1248, 2004
15. Radaev et al., Structure 11, 1527-1535, 2003

## Revendications

1. Protéine de fusion comprenant le domaine lectinique d'une galectine fusionné avec une molécule d'intérêt, avec un site de clivage par protéase inséré entre les deux, où le domaine lectinique est le domaine CRD_{SAT} de séquence SEQ ID NO : 1.

2. Protéine de fusion selon la revendication 1, où le site de clivage est un site de clivage par la protéase TEV.

3. Vecteur d'expression d'une protéine de fusion telle que définie dans l'une quelconque des revendications 1 ou 2, ledit vecteur comprenant les éléments suivants liés de manière fonctionnelle :
a) un promoteur placé en 5' des éléments b), c) et d) ;
b) une séquence codant pour le domaine CRD_{SAT} de séquence SEQ ID NO : 1 ;
c) un site de clonage recevant une séquence codant pour une protéine d'intérêt ;
d) des signaux de terminaison de la transcription ;
et dans lequel un site de clivage par protéase est localisé entre la séquence b) et le site de clonage c).

4. Vecteur d'expression selon la revendication 3, où la séquence b) est placée à proximité du site de clonage c), en 3' du promoteur.

5. Vecteur d'expression selon l'un quelconque des revendications 3 ou 4, où le site de clivage par protéase est un site de clivage par la protéase TEV.

6. Vecteur d'expression selon l'une quelconque des revendications 3 à 5, où ledit vecteur est le vecteur d'expression pCARGHO dérivé du vecteur pET20b(+) et comprenant les éléments suivants :
une origine de réplication en position 1944 ;
un promoteur T7 en position 797-813 ;
un site de fixation au ribosome (RBS) en position 742-747 ;
la séquence du CRD_{SAT} en position 242-736 ;
une séquence site de reconnaissance TEV en position 221-241 ;
un site de clonage multiple en position 159-215 ;
un terminateur T7 en position 26-72 ;
une origine F1 en position 3694-4149 ; et
un gène *bla* de résistance à l'ampicilline en position 2705-3562.

7. Procédé de production d'une protéine d'intérêt purifiée, ledit procédé comprenant :
a) la préparation d'un vecteur d'expression tel que défini dans l'une quelconque des revendications 3 à 6 ;
b) la transformation d'une cellule hôte avec ledit vecteur d'expression ;
c) la culture de ladite cellule hôte ainsi transformée dans des conditions permettant l'expression de la protéine de fusion et favorisant sa solubilisation dans les cellules hôtes ; et
d) la purification de la molécule d'intérêt.

8. Procédé selon la revendication 7, dans lequel l'étape de purification d) est réalisée par :
(i) liaison de la protéine de fusion via le domaine lectinique de la galectine ou partie dudit domaine sur un support de chromatographie greffé avec des molécules de lactose, de préférence sur une colonne de résine d'agarose ou de Sepharose greffée avec des molécules de lactose ;
(ii) clivage par protéase de la protéine d'intérêt ;
(iii) élution de la protéine d'intérêt ;
(iv) séparation de la protéase et de la protéine d'intérêt.

9. Procédé selon la revendication 7, dans lequel l'étape de purification d) est réalisée par :
(i) liaison de la protéine de fusion via le domaine lectinique de la galectine ou partie dudit domaine sur un support de chromatographie greffé avec des molécules de lactose, de préférence sur une colonne de résine d'agarose ou de Sepharose greffée avec des molécules de lactose ;
(ii) élution de la protéine de fusion ;
(iii) clivage par protéase de la protéine d'intérêt en solution ;
(iv) séparation de la protéase et du domaine lectinique ou partie dudit domaine, de la protéine d'intérêt.

10. Procédé selon l'une quelconque des revendications 8 ou 9, où l'étape de séparation (iv) est réalisée par chromatographie d'échanges d'ions ou chromatographie d'exclusion stérique ou chromatographie d'interactions hydrophobes.

11. Procédé selon l'une quelconque des revendications 8 à 10, où l'étape d'élution est réalisée avec une solution de lactose.

12. Domaine CRD_{SAT} de séquence en acides aminés SEQ ID NO : 1.

## Patentansprüche

1. Fusionsprotein, welches die lektinartige Domäne eines Galektins umfasst, wobei diese mit einem Molekül von Interesse fusioniert ist, wobei zwischen den beiden eine Spaltungsstelle für eine Protease eingefügt ist, wobei es sich bei der lektinartigen Domäne um die Domäne CRD_{SAT} mit der Sequenz SEQ ID Nr.: 1 handelt.

2. Fusionsprotein nach Anspruch 1, wobei es sich bei der Spaltungsstelle um eine Spaltungsstelle für die Protease TEV handelt.

3. Expressionsvektor für ein Fusionsprotein gemäß der Begriffsbestimmung in einem beliebigen der Ansprüche 1 oder 2, wobei der Vektor die folgenden Elemente umfasst, welche funktionsfähig verknüpft sind:
a) einen Promotor, der 5'-seitig der Elemente b), c) und d) angeordnet ist;
b) eine Sequenz, welche für Domäne CRD_{SAT} mit der Sequenz SEQ ID Nr.: 1 codiert;
c) eine Klonierungsstelle, die eine Sequenz aufnimmt, welche für ein Protein von Interesse codiert;
d) Signale, welche die Transkription beenden,
und wobei sich darin eine Spaltungsstelle für eine Protease bindet, die zwischen der Sequenz b) und der Klonierungsstelle c) angeordnet ist.

4. Expressionsvektor nach Anspruch 3, wobei die Sequenz b) in der Nähe der Klonierungsstelle c) angeordnet ist, 3'-seitig des Promotors.

5. Expressionsvektor nach einem beliebigen der Ansprüche 3 oder 4, wobei es sich bei der Spaltungsstelle für eine Protease um eine Spaltungsstelle für die Protease TEV handelt.

6. Expressionsvektor nach einem beliebigen der Ansprüche 3 bis 5, wobei es sich bei dem Vektor um den Expressionsvektor pCARGHO handelt, der sich vom Vektor pET20b(+) ableitet und die folgenden Elemente umfasst:
einen Replikationsursprung an der Position 1944;
einen Promotor T7 an den Positionen 797-813;
eine Ribosomenbindungsstelle (RBS) an den Positionen 742-747;
die Sequenz von CRD_{SAT} an den Positionen 242-736;
eine Sequenz der TEV-Erkennungsstelle an den Positionen 221-241;
eine Mehrfach-Klonierungsstelle an den Positionen 159-215;
einen Terminator T7 an den Positionen 26-72;
einen Ursprung F1 an den Positionen 3694-4149; und
ein *bla-*Gen für Ampicillin-Resistenz an den Positionen 2705-3562.

7. Verfahren zur Produktion eines aufgereinigten Proteins von Interesse, wobei das Verfahren Folgendes umfasst:
a) Herstellen eines Expressionsvektors gemäß der Begriffsbestimmung in einem beliebigen der Ansprüche 3 bis 6;
b) Transformieren einer Wirtszelle mit dem Expressionsvektor;
c) Anzüchten der auf diese Weise transformierten Wirtszelle unter Bedingungen, welche die Expression des Fusionsproteins ermöglichen und dessen Solubilisation in den Wirtszellen begünstigen; und
d) Aufreinigen des Moleküls von Interesse.

8. Verfahren nach Anspruch 7, wobei der Aufreinigungsschritt d) folgendermaßen durchgeführt wird:
(i) Binden des Fusionsproteins über die lektinartige Domäne des Galektins, oder einen Abschnitt dieser Domäne, an einen Chromatographieträger, auf welchen Lactosemoleküle gepfropft sind, vorzugsweise an eine Agarose- oder Sepharoseharzsäule, auf welche Lactosemoleküle gepfropft sind;
(ii) Abspalten des Proteins von Interesse durch eine Protease;
(iii) Eluieren des Proteins von Interesse;
(iv) Trennen der Protease und des Proteins von Interesse.

9. Verfahren nach Anspruch 7, wobei der Aufreinigungsschritt d) folgendermaßen durchgeführt wird:
(i) Binden des Fusionsproteins über die lektinartige Domäne des Galektins, oder einen Abschnitt dieser Domäne, an einen Chromatographieträger, auf welchen Lactosemoleküle gepfropft sind, vorzugsweise an eine Agarose- oder Sepharoseharzsäule, auf welche Lactosemoleküle gepfropft sind;
(ii) Eluieren des Fusionsproteins;
(iii) Abspalten des Proteins von Interesse durch eine Protease in Lösung;
(iv) Abtrennen der Protease und der lektinartigen Domäne, oder des Abschnitts dieser Domäne, vom Protein von Interesse.

10. Verfahren nach einem beliebigen der Ansprüche 8 oder 9, wobei der Trennschritt (iv) mittels lonenaustausch-Chromatographie oder sterischer Ausschlusschromatographie oder mittels Chromatographie durch hydrophobe Wechselwirkungen durchgeführt wird.

11. Verfahren nach einem beliebigen der Ansprüche 8 bis 10, wobei der Elutionsschritt mit einer Lactoselösung durchgeführt wird.

12. CRD_{SAT}-Domäne mit der Aminosäuresequenz SEQ ID Nr.: 1.

## Claims

1. Fusion protein comprising the lectin domain of a galectin fused with a molecule of interest, with a site for cleavage by protease inserted between the two, wherein the lectin domain is the CRD_{SAT} domain of sequence SEQ ID NO: 1.

2. Fusion protein according to claim 1, wherein the cleavage site is a site for cleavage by TEV protease.

3. Expression vector for a fusion protein according to either one of claims 1 or 2, said vector comprising the following functionally linked elements:
a) a promoter placed 5' of the elements b), c) and d);
b) a sequence encoding the CRD_{SAT} domain of sequence SEQ ID NO: 1;
c) a cloning site receiving a sequence encoding a protein of interest;
d) transcription termination signals;
and wherein a site for cleavage by protease is located between the sequence b) and the cloning site c).

4. Expression vector according to claim 3, wherein the sequence b) is placed near to the cloning site c), 3' of the promoter.

5. Expression vector according to either one of claims 3 or 5, wherein the site for cleavage by protease is a site for cleavage by TEV protease.

6. Expression vector according to any one of claims 3 to 5, wherein said vector is the pCARGHO expression vector derived from the pET20b(+) vector and comprising the following elements:
an origin of replication at position 1944;
a T7 promoter at position 797-813;
a ribosome binding site (RBS) at position 742-747;
the CRD_{SAT} sequence at position 242-736;
a TEV recognition site sequence at position 221-241;
a multiple cloning site at position 159-215;
a T7 terminator at position 26-72;
an F1 origin at position 3694-4149; and
a *bla* ampicillin resistance gene at position 2705-3562.

7. Method for producing a purified protein of interest, said process comprising:
a) preparing an expression vector according to any one of claims 3 to 6;
b) transforming a host cell with said expression vector;
c) culturing said host cell transformed in this way under conditions enabling the expression of the fusion protein and promoting the dissolution thereof in host cells; and
d) purifying the molecule of interest.

8. Method according to claim 7, wherein the purification step d) is carried out by:
(i) binding the fusion protein, via the lectin domain of the galectin or part of said domain, to a chromatography support grafted with lactose molecules, preferably on a column of sepharose or agarose resin grafted with lactose molecules;
(ii) cleavage by protease of the protein of interest;
(iii) elution of the protein of interest;
(iv) separation of the protease and the protein of interest.

9. Method according to claim 7, wherein the purification step d) is carried out by:
(i) binding the fusion protein, via the lectin domain of the galectin or part of said domain, to a chromatography support grafted with lactose molecules, preferably on a column of sepharose or agarose resin grafted with lactose molecules;
(ii) elution of the fusion protein;
(iii) cleavage by protease of the protein of interest in solution;
(iv) separation of the protease and the lectin domain or part of said domain from the protein of interest.

10. Method according to either one of claims 8 or 9, in which the separation step (iv) is carried out by ion exchange chromatography or size exclusion chromatography or hydrophobic interaction chromatography.

11. Method according to any one of claims 8 to 10, in which the elution step is carried out with a lactose solution.

12. CRD_{SAT} domain of amino acid sequence SEQ ID NO: 1.
